# EUROPÄISCHE PATENTSCHRIFT

(11) **EP 1 541 676 B1**
(45) Veröffentlichungstag und Bekanntmachung des Hinweises auf die Patenterteilung: **24.12.2008**
(21) Anmeldenummer: 04022637.5
(22) Anmeldetag: 23.09.2004
(51) Int. Cl.: C12N 9/74, C07K 16/40, C12N 5/20, G01N 33/68

(54) **Gegen das Prothrombin-Fragment F1+2 gerichtete Antikörper, ihre Herstellung und Verwendung**
Antibodies to fragment F1+2 of prothrombin, their production and use
Les anticorps dirigés contre le fragment F1+2 du progthrombin, leur production et utilisation

(30) Priorität: 20.11.2003 DE 10354403
(43) Veröffentlichungstag der Anmeldung: 15.06.2005
(73) Patentinhaber: Siemens Healthcare Diagnostics Products GmbH, 35041 Marburg (DE)
(72) Erfinder: Teigelkamp, Stefan, Dr., 35085 Ebsdorfergrund (DE); Braun, Konrad, 35085 Ebsdorfergrund (DE)

(56) Entgegenhaltungen:
- WO-A-00/14209
- WO-A-20/04111636
- US-B1- 6 541 275

## Beschreibung

Die Erfindung betrifft gegen das Prothrombin-Fragment F₁₊₂ gerichtete Antikörper, ihre Herstellung und Verwendung.

Die Umwandlung von Prothrombin zum aktiven Thrombin unter Bildung von Fragmenten stellt ein zentrales Ereignis im Ablauf der Gerinnungskaskade dar. Mit der immunchemischen Bestimmung des Prothrombinfragments F₁₊₂ wird eine Quantifizierung des tatsächlich gebildeten Thrombins möglich

Die Bedeutung der Bestimmung von Prothrombinfragment F₁₊₂ (nachfolgend "F₁₊₂" genannt) liegt in der Diagnose hyperkoagulatorischer Zustände bzw. thrombotischer Ereignisse. Erhöhte Werte werden bei Patienten mit Thrombosen, Lungenembolie, disseminierter intravasaler Gerinnung (DIC), Polytrauma und Sepsis nachgewiesen. Eine Erhöhung der F₁₊₂-Konzentration in Plasma von Patienten mit hereditärem Protein C- bzw. Protein-S-Mangel ist ebenfalls beschrieben. Unter der Therapie mit oralen Antikoagulantien wurde ein deutliches Absinken des F₁₊₂-Spiegels unter den Referenzbereich festgestellt.

Generell tritt bei F₁₊₂-Testen die Schwierigkeit auf, dass Prothrombin in der Probe im Vergleich zu F₁₊₂ in großem Überschuss vorliegt, so dass die im Test einzusetzenden Anti-F₁₊₂-Antikörper sehr spezifisch zwischen durch Spaltung freigesetzten Prothrombinfragmenten F₂ (nachfolgend "F₂" genannt) und F₁₊₂ einerseits und intaktem Prothrombin andererseits unterscheiden müssen. Die Herstellung solcher spezifischer Antikörper ist in EP-0 303 983 beschrieben. Ein kommerziell erhältlicher Enzymimmunoassay zur Bestimmung der Konzentration des Prothrombin-Fragmentes F₁₊₂ nutzt z.B. so hergestellte polyklonale Anti-F₁₊₂-Kaninchenantikörper. Für die Spezifität der Anti-F₁₊₂-Antikörper ist wichtig, dass diese an ein Epitop binden, welches zumindest die vier carboxyterminalen Aminosäuren der F₂- und F₁₊₂-Fragmente (Ile-Glu-Gly-Arg-OH) enthält. Obwohl auch entsprechende monoklonale Anti-F₁₊₂-Antikörper bereits seit Jahren bekannt sind (US 6541275 , EP-0 594 576) und auch die generellen Vorteile von monoklonalen Antikörpern in der Fachwelt unbestritten sind, würden diese allerdings in kommerziellen F₁₊₂-Testen bisher nicht eingesetzt. Da in der Regel zur Bestimmung der F₁₊₂-Konzentration Sandwich-Immunoassays eingesetzt werden, werden zwei Anti-F₁₊₂-Antikörper benötigt. Offensichtlich ist es bisher nicht gelungen, eine Kombination, insbesondere aus monoklonalen Antikörpern, zu finden, die es erlaubt, mit hoher Sensitivität und Spezifität F₁₊₂ zu bestimmen.

Der vorliegenden Erfindung lag demnach die Aufgabe zugrunde, einen Test zur Bestimmung des Prothrombin-Fragments F₁₊₂ mit erhöhter Präzision, Reproduzierbarkeit und verbesserter Diskriminierung von pathologischen und nicht pathologischen Proben zu Verfügung zu stellen.

Diese Aufgabe wird durch die Bereitstellung der in den Ansprüchen beschriebenen erfindungsgemäßen Verfahren und Gegenstände gelöst.

Insbesondere wird die Aufgabe durch die Bereitstellung von monoklonalen Antikörpern gegen das Prothrombin-Fragment F₁₊₂, die an ein Epitop auf der N-terminalen Hälfte des F₂-Fragmentes von Prothrombin binden, (nachfolgend "Sekundärantikörper" genannt) gelöst. Diese Sekundärantikörper bilden in Kombination mit Antikörpern, deren Epitope die vier carboxyterminalen Aminosäuren der F₂- und F₁₊₂-Fragmente umfassen, (nachfolgend "Primärantikörper" genannt), die Basis für einen verbesserten Sandwich-Immunoassay zur Bestimmung von F₁₊₂.

Es hat sich überraschenderweise im Rahmen einer Immunisierungsstudie gezeigt, dass zwar alle getesteten Primärantikörper eine Bindung an F₁₊₂ zeigten, dass aber nur vier von 38 getesteten Sekundärantikörpern spezifisch an F₁₊₂ binden. Zum Aufbau eines Sandwich-Immunoassays waren zwar 75% der getesteten Primärantikörper geeignet, aber nur einer der übriggebliebenen vier Sekundärantikörper. Diese geringe Ausbeute deutet darauf hin, dass der Sekundärantikörper über besondere Eigenschaften verfügen muss. Besonders wichtig in diesem Zusammenhang ist das vom Sekundärantikörper erkannte Epitop.

Bei einem erfindungsgemäßen Antikörper wurde das Epitop als ein Peptid mit der Aminosäuresequenz Ser-Pro-Pro-Leu-Glu-Gln-Cys eindeutig identifiziert.

Im folgenden werden spezifische Ausführungsformen der Erfindung näher erläutert:

Ein Gegenstand dieser Erfindung sind Peptide umfassend die Aminosäuresequenz Pro-Leu-Glu-Gln-Cys dadurch gekennzeichnet, dass sie die Aminosäuresequenz Ser-Glu-Gly-Ser-Ser-Val-Asn-Leu-Ser-Pro-Pro-Leu-Glu-Gln-Cys-Val-Pro-Asp-Arg-Gly-Gln-Gln-Tyr-Gln-Gly oder Ser-Pro-Pro-Leu-Glu-Gln-Cys aufweisen.

Der Begriff "Peptide" im Sinne dieser Erfindung umfasst Säureamide, die bei Hydrolyse in Aminosäuren zerfallen, beispielsweise Aminosäurepolymere wie z.B. Polypeptide, Oligopeptide, Proteine oder Proteinfragmente.

Die erfindungsgemäßen Peptide können als Immunisierungsantigen zur Herstellung der erfindungsgemäßen Antikörper oder auch zu der affinätschromatographischen Reinigung der erfindungsgemäßen Antikörper verwendet werden. Ferner können die erfindungsgemäßen Peptide auch in einem Verfahren zum quantitativen oder qualitativen Nachweis eines Analyten, bevorzugt F₁₊₂, verwendet werden. Die erfindungsgemäßen Peptide können auch mit einer Festphase und/oder einer Komponente eines signalbildenden Systems assoziiert sein.

Ein weiterer bevorzugter Gegenstand der Erfindung sind Antikörper, die dadurch gekennzeichnet sind, dass diese Antikörper spezifisch an an eines der beiden Peptide mit der Aminosäuresequenz Ser-Glu-Gly-Ser-Ser-Val-Asn-Leu-Ser-Pro-Pro-Leu-Glu-Gln-Cys-Val-Pro-Asp-Arg-Gly-Gln-Gln-Tyr-Gln-Gly oder mit der Aminosäuresequenz Ser-Pro-Pro-Leu-Glu-Gln-Cys binden.

Unter dem Begriff "Antikörper" ist im Sinne dieser Erfindung ein Immunglobulin, z.B. ein Immunglobulin der Klasse bzw. Subklasse IgA, IgD, IgE, IgG₁, IgG₂ₐ, IgG_{2b}, IgG₃, IgG₄, IgM, zu verstehen. Ein Antikörper weist mindestens eine Bindungsstelle (häufig Paratop genannt) für ein Epitop (häufig auch antigene Determinante genannt) auf einem Antigen oder Hapten auf. Ein solches Epitop ist z.B. durch seine räumliche Struktur und/oder durch das Vorhandensein von polaren und/oder apolaren Gruppen gekennzeichnet. Die Bindungsstelle des Antikörpers ist komplementär zum Epitop. Die Antigen-Antikörper-Reaktion bzw. die Hapten-Antikörper-Reaktion funktioniert nach dem sogenannten "Schlüssel-Schlüsselloch-Prinzip", und ist in der Regel in einem hohen Grad spezifisch, d.h. die Antikörper vermögen kleine Abweichungen in der Primärstruktur, in der Ladung, in der räumlichen Konfiguration und der sterischen Anordnung des Antigens oder Haptens zu unterscheiden. Insbesondere die sogenannten "complementarity determining regions" des Antikörpers tragen zur Bindung des Antikörpers an das Antigen oder Hapten bei.

Der Begriff "Antigene" umfaßt monovalente und polyvalente Antigene. Ein polyvalentes Antigen ist ein Molekül oder ein Molekülkomplex, an das/den mehr als ein Immunglobulin gleichzeitig binden kann, während bei einem monovalenten Antigen jeweils nur ein einziger Antikörper zur selben Zeit binden kann. Als Hapten wird üblicherweise ein Molekül bezeichnet, das nicht für sich allein immunogen ist, sondern das zu Immunisierungszwecken üblicherweise an einen Träger gebunden wird.

Unter dem Begriff Antikörper sind im Sinne dieser Erfindung nicht nur komplette Antikörper zu verstehen sondern ausdrücklich auch Antikörperfragmente, wie z.B. Fab, Fv, F(ab')₂, Fab'; sowie auch chimäre, humanisierte, bi- oder oligospezifische, oder "single chain" Antikörper; des weiteren auch Aggregate, Polymere und Konjugate von Immunglobulinen und/oder deren Fragmenten, sofern die Bindungseigenschaften an das Antigen oder Hapten erhalten sind. Antikörperfragmente lassen sich beispielsweise durch enzymatische Spaltung von Antikörpern mit Enzymen wie Pepsin oder Papain herstellen. Antikörperaggregate, - polymere und -konjugate können durch vielfältige Methoden generiert werden, z.B. durch Hitzebehandlung, Umsetzung mit Substanzen wie Glutaraldehyd, Reaktion mit immunglobulin-bindenden Molekülen, Biotinylierung von Antikörpern und anschließende Reaktion mit Streptavidin oder Avidin, etc..

Bei einem Antikörper im Sinne dieser Erfindung kann es sich um einen monoklonalen oder um einen polyklonalen Antikörper handeln. Der Antikörper kann nach den üblichen Verfahren hergestellt worden sein, z.B. durch Immunisierung des Menschen oder eines Tieres, wie z.B. Maus, Ratte, Meerschweichen, Kaninchen, Pferd, Schaf, Ziege, Huhn (s.a. Messerschmid (1996) BIOforum, 11:500-502), und anschließender Gewinnung des Antiserums; oder durch die Etablierung von Hybridomazellen und der anschließenden Reinigung der sekretierten Antikörper; oder durch Klonierung und Expression der Nukleotidsequenzen bzw. modifizierter Versionen davon, die die Aminosäuresequenzen kodieren, die für die Bindung des natürlichen Antikörpers an das Antigen und/oder Hapten verantwortlich sind.

Antikörper im Sinne dieser Erfindung sind Antikörper, die an das Peptid mit der Aminosäuresequenz Ser-Glu-Gly-Ser-Ser-Val-Asn-Leu-Ser-Pro-Pro-Leu-Glu-Gln-Cys-Val-Pro-Asp-Arg-Gly-Gln-Gln-Tyr-Gln-Gly oder an das Peptides mit der Aminosäuresequenz Ser-Pro-Pro-Leu-Glu-Gln-Cys spezifisch binden.

Besonders bevorzugte Antikörper im Sinne dieser Erfindung sind auch die Antikörper, die von der Hybridomazelllinie 92-195/097 produziert werden. Diese Hybridomazelllinie wurde am 15. August 2003 bei der DSMZ-Deutsche Sammlung von Mikroorganismen und Zellkulturen GmbH, Mascheroder Weg 1b, 38124 Braunschweig, Deutschland, unter der Eingangsnummer DSM ACC2607 hinterlegt.

Ein weiterer Gegenstand dieser Erfindung sind spezifische Bindungspartner, die an ein Epitop binden, das von einem erfindungsgemäßen Antikörper erkannt wird.

Unter einem "spezifischen Bindungspartner" ist ein Mitglied eines spezifischen Bindungspaars zu verstehen. Bei den Mitgliedern eines spezifischen Bindungspaars handelt es sich um zwei Moleküle, die jeweils mindestens eine zu einer Struktur des anderen Moleküls komplementäre Struktur aufweisen, wobei die beiden Moleküle sich über eine Bindung der komplementären Strukturen zu binden vermögen. Der Begriff Molekül umfaßt auch Molekülkomplexe wie z.B. Enzyme, die aus Apo- und Coenzym bestehen, Proteine, die aus mehreren Untereinheiten bestehen, Lipoproteine bestehend aus Protein und Lipiden, etc. Spezifische Bindungspartner können natürlich vorkommende aber auch z.B. mittels chemischer Synthese, mikrobiologischer Techniken und/oder gentechnologischer Verfahren hergestellte Substanzen sein. Zur Illustration des Begriffs spezifischer Bindungspartner, aber nicht als Einschränkung zu verstehend, sind z.B. zu nennen: Thyroxinbindendes Globulin, steroidbindende Proteine, Antikörper, Antigene, Haptene, Enzyme, Lektine, Nukleinsäuren, Repressoren, Oligo- und Polynukleotide, Protein A, Protein G, Avidin, Streptavidin, Biotin, Komplementkomponente C1q, nukleinsäurebindende Proteine, etc.. Spezifische Bindungspaare sind beispielsweise: Antikörper-Antigen, Antikörper-Hapten, Operator-Repressor, Nuclease-Nukleotid, Biotin-Avidin, Lektin-Polysaccharid, Steroid-steroidbindendes Protein, Wirkstoff-Wirkstoffrezeptor, Hormon-Hormonrezeptor, Enzym-Substrat, IgG-Protein A, komplementäre Oligo- oder Polynukleotide, etc..

Durch die Bereitstellung der erfindungsgemäßen Antikörper ist es dem Fachmann nun möglich, z.B. durch Kompetitionsexperimente (s.a. Peters et al. (1985) Monoklonale Antikörper, Springer Verlag, Kapitel 12.2 -"Epitop-Analyse"), andere spezifische Bindungspartner, Antikörper ausdrücklich miteingeschlossen, zu identifizieren, die an das Epitop eines erfindungsgemäßen Antikörpers binden. So lassen sich, nach dem Fachmann bekannten Techniken mittlerweile mit Hilfe von Phage Display Bibliotheken, über synthetische Peptiddatenbanken oder mittels "Recombinatorial Antibody Libraries" spezifische Bindungspartner selektieren (Larrick & Fry (1991) Human Antibodies and Hybridomas, 2:172-189)

Gegenstand dieser Erfindung ist auch ein erfindungsgemäßer Antikörper, der mit einer Festphase und/oder einer Komponente eines signalbildenden Systems assoziiert ist.

Der Begriff "Festphase" im Sinne dieser Erfindung beinhaltet einen Gegenstand, der aus porösem und/oder nicht porösem, in der Regel wasserunlöslichem Material besteht und die unterschiedlichsten Formen aufweisen kann, wie z,B. Gefäß, Röhrchen, Mikrotitrationsplatte, Kugel, Mikropartikel, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen wie z.B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere wie z.B. Zellulose, Nitrozellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Keramik; Glass; Metalle, insbesondere Edelmetalle wie Gold und Silber; Magnetit; Mischungen oder Kombinationen derselben; etc. Auch Zellen, Liposomen oder Phospholipidvesikel, sind vom Begriff Festphase miterfasst.

Die Festphase kann einen Überzug aus einer oder mehreren Schichten aufweisen, z.B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern, oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkender Agenzien.

Bei einem "signalbildenden System" kann es sich um eine oder mehrere Komponenten handeln, wobei es sich wenigstens bei einer Komponente um ein nachweisbares Label handelt. Als Label ist jedes Molekül zu verstehen, das selbst ein Signal produziert oder die Produktion eines Signals induzieren kann wie z.B. eine fluoreszierende Substanz, eine radioaktive Substanz, ein Enzym, oder eine chemilumineszierende Substanz. Das Signal kann beispielsweise anhand der Enzymaktivität, der Lumineszenz, der Lichtabsorption, der Lichtstreuung, der ausgestrahlten elektromagnetischen oder radioaktiven Strahlung, oder einer chemischen Reaktion nachgewiesen oder gemessen werden.

Ein Label vermag selbst ein nachweisbares Signal zu erzeugen, so dass keine weiteren Komponenten notwendig sind. Viele organische Moleküle absorbieren ultraviolettes und sichtbares Licht, wobei durch die Lichtabsorption übertragene Energie diese Moleküle in einen angeregten Energiezustand kommen können, und die absorbierte Energie in Form von Licht einer anderen Wellenlänge als der des eingestrahlten Lichts abgeben. Wieder andere Label können direkt ein nachweisbares Signal erzeugen wie z.B. radioaktive Isotope oder Farbstoffe.

Wieder andere Label benötigen zur Signalerzeugung weitere Komponenten, d.h. das signalproduzierende System schließt in einem solchen Fall all die für die Signalbildung benötigten Komponenten mit ein wie z.B. Substrate, Coenzyme, Quencher, Beschleuniger, zusätzliche Enzyme, Substanzen die mit Enzymprodukten reagieren, Katalysatoren, Aktivatoren, Cofaktoren, Inhibitoren, Ionen etc..

Geeignete Label (s.a. EP-A2-0 515 194; US 5,340,716; US 5,545,834; Bailey et al. (1987) J. Pharmaceutical & Biomedical Analysis 5:649-658) sind beispielsweise Enzyme einschließlich Meerrettichperoxidase, alkalische Phosphatase, Glukose-6-Phosphatdehydrogenase, Alkoholdehydrogenase, Glucoseoxidase, β-Galactosidase, Luciferase, Urease und Acetylcholinesterase; Farbstoffe; fluoreszierende Substanzen einschließlich Fluoresceinisothiocyanat, Rhodamin, Phycoerythrin, Phycocyanin, Ethidiumbromid, 5-Dimethylaminonaphthalen-1-sulfonylchlorid und fluoreszierende Chelate von seltenen Erden; chemilumineszierende Substanzen einschließlich Luminol, Isoluminol, Acridiniumverbindungen, Olefin, Enolether, Enamin, Arylvinylether, Dioxen, Arylimidazol, Lucigenin, Luciferin und Aequorin; Sensitizer einschließlich Eosin, 9,10-Dibromoanthracen, Methylen Blau, Porphyrin, Phthalocyanin, Chlorophyll, Rose Bengal; Coenzyme; Enzymsubstrate; radioaktive Isotope einschließlich ¹²⁵I, ¹³¹I, ¹⁴C, ³H, ³²P, ³⁵S, ¹⁴C, ⁵¹Cr, ⁵⁹Fe, ⁵⁷Co und ⁷⁵Se; Partikel einschließlich magnetische Partikel oder Partikel, bevorzugt Latexpartikel, die selbst beispielsweise mit Farbstoffen, Sensitizern, fluoreszierenden Substanzen, chemilumineszierenden Substanzen, Isotopen oder anderen nachweisbaren Labeln markiert sein können; Solpartikel einschließlich Gold- oder Silbersolen; Liposomen oder Zellen, die selbst mit nachweisbaren Labeln markiert sein können; etc.

Ein signalbildendes System kann auch Komponenten umfassen, die bei räumlicher Nähe miteinander in eine nachweisbare Wechselwirkung treten können, z.B. in Form von Energiespendern und Energieempfängern wie beispielsweise Photosensitizer und chemolumineszierende Substanzen (EP-A2-0 515 194), Photosensitizer und Fluorophore (WO 95/06877), radioaktives lod-125 und Fluorophore (Udenfriend et al. (1985) Proc. Natl. Acad. Sci. 82:8672-8676), Fluorophore und Fluorophore (Mathis (1993) Clin. Chem. 39:1953-1959) oder Fluorophore und Fluoreszenz-Quencher (US 3,996,345).

Unter einer Wechselwirkung zwischen den Komponenten ist die direkte Übertragung von Energie zwischen den Komponenten, z.B. durch Licht- oder Elektronenstrahlung sowie über kurzlebige reaktive chemische Moleküle, miteingeschlossen. Ferner umfasst sind hiervon auch Vorgänge, bei denen die Aktivität einer Komponente durch eine oder mehrere andere inhibiert oder verstärkt wird, beispielsweise die Hemmung oder Steigerung der Enzymaktivität oder die Hemmung, Steigerung oder Veränderung (z.B. Wellenlängenverschiebung, Polarisation) des von der beeinflussten Komponente ausgesendeten elektromagnetischen Strahlung. Die Wechselwirkung zwischen den Komponenten umfasst auch Enzymkaskaden. In diesem Fall sind die Komponenten Enzyme, von denen mindestens eines das Substrat für ein anderes liefert, so dass eine maximale oder minimale Reakionsgeschwindigkeit der gekoppelten Substratumsetzung resultiert.

Eine effektive Wechselwirkung zwischen den Komponenten findet in der Regel statt, wenn diese räumlich benachbart vorliegen, also z.B. innerhalb eines Abstandbereiches von wenigen µm, insbesondere innerhalb eines Abstandbereiches von unter 600 nm, bevorzugt unter 400 nm, ganz besonders bevorzugt von unter 200 nm.

Mikropartikel werden häufig als Festphase und/oder als Label genutzt. Unter dem Begriff "Mikropartikel" sind im Sinne dieser Erfindung Teilchen zu verstehen, die einen ungefähren Durchmesser von wenigstens 20 nm und nicht mehr als 20 µm aufweisen, üblicherweise zwischen 40 nm und 10 µm, bevorzugt zwischen 0,1 und 10 µm, besonders bevorzugt zwischen 0,1 und 5 µm, ganz besonders bevorzugt zwischen 0,15 und 2 µm. Die Mikropartikel können regelmäßig oder unregelmäßig geformt sein. Sie können Kugeln, Spheroide, Kugeln mit mehr oder weniger großen Kavitäten oder Poren darstellen. Die Mikropartikel können aus organischem, aus anorganischem Material oder aus einer Mischung oder Kombination von beiden bestehen. Sie können aus einem porösen oder nicht porösen, einem schwellfähigen oder nicht schwellfähigen Material bestehen. Prinzipiell können die Mikropartikel jegliche Dichte haben, bevorzugt sind jedoch Partikel mit einer Dichte, die der Dichte des Wassers nahe kommt wie etwa 0,7 bis etwa 1,5 g/ml. Die bevorzugten Mikropartikel sind in wässrigen Lösungen suspendierbar und möglichst lange suspensionsstabil. Sie mögen durchsichtig, teilweise durchsichtig oder undurchsichtig sein. Die Mikropartikel können aus mehreren Schichten bestehen wie beispielsweise die sogenannten "core-and-shell" Partikel mit einem Kern und einer oder mehreren umhüllenden Schichten. Der Begriff Mikropartikel umfasst beispielsweise Farbstoffkristalle, Metallsolen, Silica-Partikel, Glaspartikel, Magnetpartikel, Polymerteilchen, Öltropfen, Lipidpartikel, Dextran, und Proteinaggregate. Bevorzugte Mikropartikel sind in wässrigen Lösungen suspendierbare und aus wasserunlöslichem Polymermaterial bestehende Partikel, insbesondere aus substituierten Polyethylenen. Ganz besonders bevorzugt sind Latexpartikel z.B. aus Polystyrol, Acrylsäurepolymeren, Methacrylsäurepolymeren, Acrylnitril-Polymeren, Acrylnitril-Butadien-Styrol, Polyvinylacetat-Acrylat, Polyvinylpyridin, Vinylchlorid-Acrylat. Von besonderem Interesse sind Latexpartikel mit reaktiven Gruppen an ihrer Oberfläche wie beispielsweise Carboxyl-, Amino- oder Aldehydgruppen, die eine kovalente Bindung z.B. von spezifischen Bindungspartnern an die Latexpartikel erlauben. Die Herstellung von Latexpartikeln ist beispielsweise in EP 0 080 614, EP 0 227 054 und EP 0 246 446 beschrieben.

Der Begriff "assoziiert" ist breit zu verstehen und umfasst beispielsweise eine kovalente und eine nicht-kovalente Bindung, eine direkte und eine indirekte Bindung, die Adsorption an eine Oberfläche und den Einschluß in eine Vertiefung oder einen Hohlraum, etc.. Bei einer kovalenten Bindung sind die Antikörper oder Bindungspartner über eine chemische Bindung an die Festphase oder an das Label gebunden. Beispiele für eine nicht-kovalente Bindung sind die Oberflächenadsorption, der Einschluss in Hohlräume oder die Bindung von zwei spezifischen Bindungspartnern. Neben einer direkten Bindung an die Festphase oder das Label können die Antikörper oder Bindungspartner auch indirekt über spezifische Wechselwirkung mit anderen spezifischen Bindungspartnern an die Festphase oder das Label gebunden sein (s.a. EP-A2-0 411 945). Dies soll anhand von Beispielen näher illustriert werden: Der biotinylierte Antikörper kann über labelgebundenes Avidin an das Label gebunden werden; oder es kann ein Fluorescein-Antikörperkonjugat über festphasengebundene Anti-Fluorescein-Antikörper an die Festphase gebunden werden; oder der Antikörper kann über Immunglobulin-bindende Proteine an die Festphase oder das Label gebunden werden.

Ein weiterer Gegenstand dieser Erfindung sind erfindungsgemäße Antikörper oder spezifische Bindungspartner die als ein in vitro Diagnostikum oder als ein Bestandteil eines in vitro Diagnostikums verwendet werden.

Bei einem in vitro Diagnostikum wird der nachzuweisende Analyt, z.B. F₁₊₂, in einer Probe außerhalb eines lebenden menschlichen oder tierischen Körpers nachgewiesen oder dessen Konzentration oder Menge bestimmt.

Unter einer "Probe" ist im Sinne der Erfindung das Material zu verstehen, das die nachzuweisende Substanz (Beispiele für den Begriff "Analyt" siehe EP-A2-0 515 194, Seiten 8-15) vermutlich enthält. Der Begriff Probe umfasst beispielsweise biologische Flüssigkeiten oder Gewebe insbesondere von Menschen und Tieren wie Blut, Plasma, Serum, Sputum, Exudat, bronchoalveoläre Lavage, Lymphflüsigkeit, Synovialflüssigkeit, Samenflüssigkeit, Vaginalschleim, Feces, Urin, Liquor, Haare, Haut, Gewebeproben oder -schnitte. Ferner werden umfasst Zellkulturproben, pflanzliche Flüssigkeiten oder Gewebe, forensische Proben, Wasser- und Abwasserproben, Nahrungsmittel, Arzneimittel. Gegebenenfalls müssen die Proben vorbehandelt werden, um den Analyten für das Nachweisverfahren zugänglich zu machen oder um störende Probenbestandteile zu entfernen. Solche Vorbehandlung von Proben mag die Abtrennung und/oder Lyse von Zellen beinhalten, das Präzipitieren, die Hydrolyse oder die Denaturierung von Probenbestandteilen wie z.B. Proteinen, die Zentrifugation von Proben, das Behandeln der Probe mit organischen Lösungsmitteln wie z.B. Alkohole, insbesondere Methanol; das Behandeln der Probe mit Detergenzien. Häufig wird die Probe in ein anderes, meistens wässriges, Medium überführt welches mit dem Nachweisverfahren möglichst nicht interferieren soll.

Die erfindungsgemäßen Antikörper können in einem Verfahren zum quantitativen oder qualitativen Nachweis eines Analyten, bevorzugt F₂ und/oder F₁₊₂ und/oder Prothrombin in einer Probe verwendet werden.

Bei einem quantitativen Nachweis wird die Menge, die Konzentration oder die Aktivität (z.B. Enzymaktivität) des Analyten in der Probe gemessen. Von dem Begriff des "quantitativen Nachweises" sind auch semiquantitative Methoden umfasst, die nur die ungefähre Menge, Konzentration oder Aktivität des Analyten in der Probe erfassen oder nur zu einer relativen Mengen-, Konzentrations- oder Aktivitätsangabe dienen können. Unter einem qualitativen Nachweis ist der Nachweis des Vorhandenseins des Analyten in der Probe überhaupt oder das Anzeigen, dass die Konzentration oder Aktivität des Analyten in der Probe unterhalb oder oberhalb eines bestimmten oder mehrerer bestimmter Schwellenwerte liegt, zu verstehen.

Die Erfindung betrifft somit auch Verfahren zum quantitativen oder qualitativen Nachweis eines Analyten, bevorzugt F₂ und/oder F₁₊₂, in einer Probe und geeignete Reagenzien hierfür.

Zum Nachweis von Analyten werden häufig Bindungsteste eingesetzt, bei denen durch eine spezifische Bindung von nachzuweisenden Analyt an analytspezifische Bindungspartner auf die Anwesenheit, Abwesenheit oder Menge des Analyten in einer Probe geschlossen werden kann. Immunoassays oder auch Verfahren, bei denen Oligo- oder Polynukleotide hybridisiert werden, sind Beispiele für Bindungsteste.

Die sogenannten "heterogenen Bindungsteste" sind durch einen oder mehrere Trennungsschritte und/oder Waschschritte gekennzeichnet. Die Trennung kann beispielsweise durch Immunfällung, Fällung mit Substanzen wie Polyethylenglykol oder Ammoniumsulfat, Filtration, magnetische Abtrennung, Anbindung an eine Festphase erfolgen. Ein solche "Festphase" besteht aus porösem und/oder nicht porösem, in der Regel wasserunlöslichem Material. Sie kann die unterschiedlichsten Formen aufweisen wie z.B.: Gefäß, Röhrchen, Mikrotitrationsplatte, Kugel, Mikropartikel, Stäbchen, Streifen, Filter- oder Chromatographiepapier, etc. Bei heterogenen Bindungstesten im Sandwichformat ist in der Regel einer der analytspezifischen Bindungspartner an eine Festphase gebunden und dient zur Abtrennung des Bindungskomplexes "Analyt/analytspezifischer Bindungspartner" von der flüssigen Phase, während der andere analytspezifische Bindungspartner zum Nachweis des Bindungskomplexes ein nachweisbares Label (z.B. ein Enzym, ein Fluoreszenz- oder Chemilumineszenzlabel, etc.) trägt. Man unterteilt diese Testverfahren weiter in sogenannte Einschritt-Sandwich-Teste, bei denen die beiden spezifischen Bindungspartner simultan mit der Probe inkubiert werden und in Zweischritt-Sandwich-Teste, bei denen die Probe zunächst mit dem Festphasenreagenz inkubiert wird und nach einem Trenn- und Waschschritt der festphasengebundene Bindungskomplex aus Analyt und analytspezifischer Bindungspartner mit dem Nachweisreagenz inkubiert wird.

Bei "homogenen Bindungstesten" erfolgt keine Trennung zwischen freien und an den "Analyt/analytspezifischer Bindungspartner"-Komplex gebundenen Komponenten des signalbildenden Systems. Der Testansatz, welcher die analytspezifischen Bindungspartner, die signalbildenden Komponenten und die Probe enthält, wird nach oder sogar während der Bindungsreaktion ohne einen weiteren Trenn- und/oder Waschschritt gemessen und das entsprechende Messsignal bestimmt. Beispiele für homogene Immunoassays (s.a. Boguslaski & -Li- (1982) Applied Biochemistry and Biotechnology, 7:401-414) sind viele turbidimetrische oder nephelometrische Methoden, wobei die zum Nachweis verwendeten analytspezifischen Bindungspartner mit Latexpartikel assoziiert sein können; EMIT^{®}-Teste; CEDIA^{®}-Teste; Fluoreszenz-Polarisations-Immunoassays; Luminescent Oxygen Channeling Immunoassays ("LOCI", s. EP-A2-0 515 194; Ullman et al. (1994) Proc. Natl. Acad. Sci., 91:5426-5430; Ullman et al. (1996) Clinical Chemistry ,42:1518 - 1526); etc.. Bei einem homogenen Sandwich-Immunoassay, wie z.B. einem nephelometrischen Latextest, werden die Antikörperreagenzien mit der Probe zusammen inkubiert und die Messung des Signals während und/oder nach der Inkubation durchgeführt, ohne dass ein Trenn- oder Waschschritt vor der Messung durchgeführt wird. Mit anderen Worten ausgedrückt: Es folgt keine Trennung des Antikörper-gebundenen Analyten vom freien Analyten oder von Antikörpern, die keinen Analyten gebunden haben.

Homogene und heterogene Bindungsteste können auch in Form eines sogenannten "Sandwichassays" durchgeführt werden. Hierbei wird der Analyt z.B. bei einem heterogenen Bindungstest von einem Festphasen-assoziierten analytspezifischen Bindungspartner und einem analytspezifischen Bindungspartner, der mit einer Komponente eines signalbildenden System assoziiert ist, gebunden. Bei Sandwichimmunoassays können Antikörper oder Antigene oder Haptene die analytspezischen Bindungspartner bilden.

Eine weitere spezielle Ausführungsform eines heterogenen oder homogenen Bindungstestes ist der "indirekte Immunoassay". Der Analyt ist in diesem Fall ein Antikörper. Einer der analytspezifischen Bindungspartner ist das Antigen oder ein modifiziertes Antigen des nachzuweisenden Antikörpers (= Analyt) und der andere analytspezifische Bindungspartner ist in der Regel ein Immunglobulin-bindendes Protein wie z.B. ein Antikörper, der den nachzuweisenden Antikörper (= Analyt) spezifisch zu binden vermag.

Bei einem homogenen oder heterogenen "kompetitiven Bindungstest" konkurrieren Proben-Analyt und Reagenz-Analyt (beispielsweise ein "modifizierter Analyt" wie z.B. ein gelabelter oder markierter Analyt, Analytteilstück oder Analytanalogon), um die Bindung an eine limitierte Anzahl von analytspezifischen Bindungspartnern. Beispiele zur illustration des Prinzips: (i) Proben-Analyt konkurriert mit Reagenz-Analyt, der mit einer Komponente eines signalbildenden System assoziiert ist, um die Bindung an Festphasen-assoziierte analytspezifische Bindungspartner oder (ii) Proben-Analyt konkurriert mit Festphasen-assoziiertem Analyt (= Reagenz-Analyt) um die Bindung an analytspezifische Bindungspartner, die mit einer Komponente eines signalbildenden Systems assoziiert sind.

Der Nachweis von F₁₊₂ mit den erfindungsgemäßen Antikörpern kann beispielsweise auch mit Verfahren erfolgen wie beispielsweise: Western Blot, Dot Blot, Immunelektrophorese, Immunfixations-Elektrophorese, Elektroimmundiffusion, Immunpräzipitation, radiale Immundiffusion, Immunfixation, Immunchromatographie, Latex-Agglutination, turbidimetrischer oder nephelometrischer Test, homogener oder heterogener Bindungstest, Ein- oder Zweischritt-Test, Sandwich-Test, indirekter Test, kompetitiver Test, "point-of-care"-Teste, etc.. Diese und andere Nachweisverfahren sind beispielsweise in "Labor und Diagnose", ed. L. Thomas, TH-Books Verlagsgesellschaft mbH, Frankfurt, 1998, Kapitel 60, oder in "Laboratory Techniques in Biochemistry and Molecular Biology - An Introduction to Radioimmunoassay and Related Techniques", ed. T. Chard, Elsevier, Amsterdam, 1987, beschrieben.

Der Begriff "point-of-care-Teste" oder "POC-Teste" schließt Teste ein, bei denen kein separates Analyse- oder Messgerät zur Testdurchführung oder Testauswertung benötigt wird. POC-Teste basieren in vielen Fällen auf immunchromatographische Verfahren, Immunkomplexabtrennungen per Filtration und/oder Immunfixationstechniken. Die POC-Teste sind insbesondere für Messungen vor Ort, z.B. am Krankenbett oder daheim, für den Notarzt und/oder beim niedergelassenen Arzt und weniger für das Großlabor gedacht. POC-Teste können insbesondere auch von Personen, die keine eingehende medizinisch-technische Ausbildung und Erfahrung auf dem Gebiet der Laboratoriumsmedizin haben, durchgeführt werden. Unter der Begriff "POC-Teste" sind im Sinne dieser Erfindung auch sogenannte Heimteste oder OTC-Teste zu verstehen, die von medizinischen Laien durchgeführt werden dürfen, so z.B. die diversen Schwangerschaftsteste die für den Heimgebrauch vertrieben werden. Andere POC-Teste betreffen beispielsweise den Nachweis von Herzinfarktmarkern, Drogen, Arzneimitteln, Infektions- und Entzündungsmarkern. Bei vielen POC Testen sind oder werden im Laufe der Testdurchführung spezifische Bindungspartner an oder auf Filter- oder Chromatographiestreifen oder -scheiben assoziiert. Eine positive oder negative Nachweisreaktion kann zum Beispiel mit dem Erscheinen oder Nichterscheinen einer Farbbande in einem bestimmten Testfeld verknüpft sein und/oder dem Erscheinen oder Nichterscheinen eines bestimmten Symbols, z.B. einem "+",einem "-" und/oder der Intensität des jeweiligen Meßsignals.

Ein POC-Test für F₁₊₂ kann beispielsweise so aufgebaut sein: Probe und gelabelte Antikörper, die an F₁₊₂ zu binden vermögen (bevorzugt Sekundärantikörper), werden auf einen Teststreifen aufgetragen. Geeignete Label sind z.B. gefärbte Latexpartikel, kolloidales Gold, Enzyme, etc.. Sofern F₁₊₂ in der Probe enthalten ist, werden sich F₁₊₂/Antikörper-Komplexe ausbilden. Diese Komplexe bewegen sich z.B. mittels Kapillarkraft in Richtung auf einen Bereich, in dem Antikörper, die an andere F₁₊₂-Epitope zu binden vermögen (bevorzugt Primärantikörper), z.B. in Form einer Bande fixiert sind oder im Laufe des Testverfahrens fixiert werden (z.B. über eine Biotin-Avidin-Brücke). Die gelabelten F₁₊₂/Antikörper-Komplexe werden in diesem Bereich gebunden und bilden mit den fixierten Antikörpern einen Sandwichkomplex aus. Die Intensität des Labelsignals ist hier proportional zur F₁₊₂-Probenkonzentration. Bei einem kompetitiven POC-Testverfahren können beispielsweise F₁₊₂ und/oder F₁₊₂-Fragmente in einem Bereich des Teststreifens fixiert sein oder im Laufe des Testverfahrens fixiert werden. Dieses fixierte F₁₊₂ würde mit F₁₊₂ aus der Probe um die Bindung an gelabelte Anti-F₁₊₂-Antikörper kompetitieren. Alternativ können auch fixierte Anti-F₁₊₂-Antikörper und gelabeltes F₁₊₂ für den Aufbau eines kompetitiven F₁₊₂-Testes eingesetzt werden.

Eine besonders bevorzugte Ausführungsform des erfindungsgemäßen Verfahrens ist ein nephelometrischer oder ein turbidimetrischer Test, insbesondere ein solcher Test bei dem erfindungsgemäße Antikörper - bevorzugt an Mikropartikel (insbesondere an Latexpartikel) assoziiert - eingesetzt werden.

Ein anderer erfindungsgemäßer Gegenstand ist ein Testkit, der einen oder mehrere der erfindungsgemäßen Antikörper und/oder Peptide enthält. In einem solchen Kit sind üblicherweise alle oder nur einige Komponenten eines Testes in abgepackter Form enthalten. Die erfindungsgemäßen Antikörper und/oder Peptide können beispielsweise mit einer oder mehreren Festphasen und/oder einer oder mehreren Komponenten eines signalbildenden Systems assoziiert sein. Der Testkit kann beispielsweise Standards; Kontrollen; sowie andere Reagenzien, wie z.B. Puffer, Waschlösungen, Messsignal-auslösende Lösungen und/oder Enzymsubstrat; Küvetten; Pipetten und/oder Testanweisungen enthalten. Eine besonders bevorzugter erfindungsgemäßer Testkit enthält an Latexpartikel assoziierte erfindungsgemäße Antikörper und/oder erfindungsgemäße Peptide.

Die erfindungsgemäßen Antikörper und Peptide lassen sich auch für die Affinitätschromatographie verwenden. Unter dem Begriff "Affinitätschromatographie" ist eine Methode zur Reinigung und Isolierung von Substanzen, insbesondere Biopolymeren, zu verstehen, die auf der Tatsache beruht, dass viele Substanzen mit für sie spezifischen Bindungspartnern eine selektive, nichtkovalente, reversible Bindung eingehen können. Das Prinzip des Verfahrens besteht darin, dass der spezifische Bindungspartner an eine unlösliche Matrix (z.B. poröse Gläser, Gele auf Agarose-, Cellulose-, Dextran-, Polymer- und Kieselgelbasis) in der Regel kovalent gebunden und in Kontakt mit einer die Substanz enthaltenden Probe gebracht wird. Die gesuchte Substanz wird wegen ihrer spezifischen Wechelswirkung mit dem Matrix-gebundenen spezifischen Bindungspartner immobilisiert und zurückgehalten, während alle anderen in der Probe enthaltenen Substanzen durch Elution abgetrennt werden. Anschließend wird die gesuchte Substanz mit einem geeigneten Elutionsmittel, das die nichtkovalente Bindung zwischen Substanz und spezifischem Bindungspartner aufhebt, von der Matrix abgelöst (s.a. E. Buddecke (1989) Grundrisse der Biochemie, Walter de Gruyter, Kapitel 7 "Proteine").

Ein anderer Gegenstand dieser Erfindung umfasst erfindungsgemäße Antikörper oder erfindungsgemäße Peptide in einem pharmazeutisch verträglichen, sterilen Injektionsmedium. Unter einem pharmazeutisch verträglichen, sterilen Injektionsmedium ist beispielsweise eine keimfreie, pyrogenfreie Lösung, z.B. Saline oder eine andere Elektrolytlösung, zu verstehen wie sie üblicherweise zur intravenösen, intramuskulären, intraperitonealen oder subkutanen Verabreichung von Arzneimitteln, Impfstoffen oder Kontrastmitteln verwendet wird.

Wieder ein anderer Gegenstand dieser Erfindung ist die Verwendung der erfindungsgemäßen Antikörper als in-vitro Diagnostikum oder als Bestandteil eines in-vitro Diagnostikums.

Je nach gewünschtem Verwendungszweck ist es vorteilhaft nur Teile der Antikörper, wie z.B. Fab-, F(ab')₂-, oder Fab'-Fragmente, einzusetzen. Diese können beispielsweise mit den dem Fachmann bekannten enzymatischen Spaltverfahren (s.a. z.B. Harlow & Lane (1988) Antibodies: A Laboratory Manual, Cold Spring Harbor Laboratory, Cold Spring Harbor) erzeugt werden.

Die Antigenbindungsstellen eines Antikörpers befinden sich in den sogenannten variablen Domänen, die durch die V-Gene kodiert sind. Mit den bekannten gentechnischen Methoden (s. z.B. Sambrook et al. (1989) Molecular Cloning: A Laboratory Manual, Cold Spring Harbor Laboratory, Cold Spring Harbor, 2nd edition; McCafferty et al. (1990) Nature 348:552-554) kann auch die entsprechende Nukleinsäuresequenz eines erfindungsgemäßen Antikörpers ermittelt werden sowie dadurch auch die entsprechende Aminosäuresequenz, sofern diese noch nicht per Aminosäuresequenzierung bereits bekannt war. Als Ausgangsmaterial für derartige Analysen können die Hybridomazellen bzw. die Antikörper produzierenden Immunzellen immunisierter Tiere eingesetzt werden.

In Kenntnis der Nuklein- und/oder Aminosäuresequenz können mit Hilfe üblicher gentechnologischer und molekularbiologischer Methoden (s.a. Johnson & Chiswell (1993) Current Opinion in Structural Biology, 3:564-571) dann humanisierte, chimäre, bi-oder oligospezifische Antikörper sowie von der "complementarity determining region" abgeleitete Peptide ("minimal recognition units"), single-chain Fragmente, und/oder funktionelle Fusionsprodukte, z.B. rekombinant hergestellte Antikörper-Enzym-Konstrukte, hergestellt werden (s. z.B. Larrick & Fry (1991) Human Antibodies and Hybridomas, 2:172-189; Kitano et al (1986) Appl. Microbiol. Biotechnol, 24:282-286; Thompson et al. (1986) J. Immunol. Methods, 94 7-12), die an ein Epitop auf der N-terminalen Hälfte des F₂-Fragmentes von Prothrombin, insbesondere an ein erfindungsgemäßes Peptid, binden. Mit solchen unter dem Begriff "Antikörper" eingeschlossenen Peptiden kann beispielsweise eine Verringerung der Immunogenität und/oder eine verstärkte Wirksamkeit bei Verabreichnung als Arzneimittel oder in vivo Diagnostikum erzielt werden und/oder es ergeben sich Vorteile für den Einsatz als oder in einem in vitro Diagnostikum. Die Antikörper sind auch herstellbar ggf. unter Zuhilfenahme gentechnologischer Methoden in pflanzlichen - wie z.B. Hefezellen - (Fischer et al. (1999) Biol. Chem., 380:825-839; Hiatt et al. (1992) Genetic Engineering, 14:49-64)), tierischen und prokaryontischen Zellen (s. z.B. WO 95/25172) sowie isolierten menschlichen Zellen.

Ein weiterer Gegenstand dieser Erfindung sind auch tierische, pflanzliche oder prokaryontische Zellen sowie isolierte menschliche Zellen, die einen erfindungsgemäßen Antikörper produzieren. Eine bevorzugte Ausführungsform dieser Erfindung umfaßt Hybridomazellinien, die die erfindungsgemäßen Antikörper produzieren, beispielsweise die Hybridomazelllinie 92-195/097. Diese Hybridomazelllinie wurde bei der DSMZ Deutsche Sammlung von Mikroorganismen und Zellkulturen GmbH, Mascheroder Weg 1b, 38124 Braunschweig, Deutschland, mit der Eingangsnummer DSM ACC2607 hinterlegt.

Die im folgenden beschriebenen Beispiele dienen zur exemplarischen Beleuchtung einzelner Aspekte dieser Erfindung und sind nicht als Einschränkung zu verstehen.

### Beispiele:

### Beispiel 1: Reinigung von Prothrombin

Als Ausgangsmaterial zur Herstellung des Immunisierungsantigens können kommerziell erhältliche Prothrombin-Präparationen eingesetzt werden. Sollten diese Produkte keine ausreichende Reinheit aufweisen, sollte das Prothrombin chromatographisch isoliert werden, bevor es zur Immunisierung eingesetzt wird. Eine chromatographische Reinigung von Prothrombin kann z.B. folgendermaßen aussehen:
1. Fraktionierung über einen Anionenaustauscher (MonoQ-Sepharose) in Citratpuffer (25 mM, pH 6) und Verwendung der Fraktionen, die Prothrombin-Aktivität zeigen. Faktor X wird durch diesen Schritt abgereichert. Elution mit 1 M NaCl.
2. Fraktionierung über Heparin-Sepharose in Citratpuffer (25 mM, pH 6) und Verwendung der Fraktionen, die Prothrombin-Aktivität zeigen. Faktor X wird durch diesen Schritt weiter abgereichert. Elution mit 1 M NaCl.
3. Entfernung von Protein C durch Affinitätschromatographie in Citratpuffer (25 mM, pH 6) mit einem monoklonalen Antikörper gegen Protein C. Der Prothrombin-haltige Durchlauf wird weiterverwendet.
4. Umpufferung in 50 mM Citrat / 150 mM NaCl, pH 6 durch Gelchromatographie mit Sephadex G200.
5. Entfernung von restlichem Faktor X durch Affinitätsreinigung im Batchverfahren in Citratpuffer (25 mM, pH 6) mit einem monoklonalen Antikörper gegen Faktor X. Der Prothrombin-haltige Überstand wird zur Immunisierung verwendet.

### Beispiel 2: Herstellung von monoklonalen Antikörpern

### A) monoklonaler Primärantikörper

Der monoklonale Primärantikörper kann gemäß den in EP-0303983und US 6,541,275 beschriebenen Verfahren hergestellt werden.

### B) monoklonaler Sekundärantikörper

Zur Herstellung der Sekundärantikörper wurde gereinigtes Prothrombin (siehe Beispiel 1) als Immunisierungsantigen verwendet (20 µg pro Maus).

### a) Immunisierung von Mäusen

BALB/c Mäuse wurden jeweils mit 20 µg Immunisierungsantigen (Prothrombin) in komplettem Freund'schen Adjuvans intraperitoneal immunisiert. Nach 4 Wochen erfolgte ein Booster mit jeweils 20 µg Immunisierungsantigen in inkomplettem Freund'schen Adjuvans (Fa. ICN Biomedical GmbH, Eschwege, Deutschland) und nach 8 Wochen mit jeweils 20 µg Immunisierungsantigen ohne Freund'sches Adjuvans. Die letzten 3 Tage vor der Fusion wurden die Mäuse intravenös mit jeweils 20 µg Immunisierungsantigen geboostert.

### b) Fusion

Nach dem Töten der Mäuse durch CO₂-Inhalation wurden die Milzen entnommen und Einzelzellsuspensionen in serumfreien Dulbeccos modifiziertem Eagle Medium (DMEM, Fa. CC Pro GmbH, Neustadt/W, Deutschland) hergestellt. Die Zellen wurden zentrifugiert (652 g) und 2 x in DMEM gewaschen. Anschließend wurde die Zellzahl mittels Trypanblau-Färbung bestimmt. Zu etwa 10⁸ Milzzellen wurden 2x10⁷ Myelomzellen (Sp2/0) gegeben. Nach dem Zentrifugieren (360 g) wurde der Überstand verworfen, 1ml Polyethylenglycol-Lösung (PEG 4000, Fa. Merck Eurolab, Bruchsal, Deutschland; ca. 50%ig in DMEM) auf das Zellpellet gegeben und nach Resuspension 1 Minute bei 37°C inkubiert. Anschließend wurde tropfenweise ca. 10 ml DMEM zugegeben und 2 bis 4 Minuten bei Raumtemperatur inkubiert. Die fusionierten Zellen wurden abzentrifugiert (326 g) und das Pellet in DMEM + 20% FKS (fötales Kälberserum, Fa. Bio Whittaker Europe, Verviers, Belgien) + HAT-Lösung (Fa. CC Pro GmbH, Neustadt/W, Deutschland) resuspendiert und in 24 Well-Zellkulturplatten (Fa. Costar) abgefüllt. Die ungefähre Zellkonzentration pro Well betrug 5x10⁴ bis 5x10⁶ Zellen.

2 - 3 Wochen später wurden die entstandenen Zellkolonien (Hybride) entnommen und in neue Kulturplatten überführt.

### c) Screening

Die Spezifität der in die Zellkultur abgegebenen Antikörper wurde in einem ersten Testschritt mit Hilfe von Prothrombin-beschichteten Mikrotiterplatten (Fa. Nunc, Typ B), Beschichtung 1 µg/ml ≈ 0,15 µg/Vertiefung, getestet.

In jede Vertiefung der Mikrotitterplatte wurden 100 µl Zellkulturüberstand (Verdünnung 1:2) pipettiert und 1 Stunde bei +15 bis +25°C inkubiert. Nach zweimaligen Waschen der Platte mit Waschlösung-POD (OSEW; Fa. Dade Behring, Marburg, Deutschland) wurden in jede Vertiefung 100 µl anti-Maus IgG/F(ab')₂-POD-Konjugat (Fa. Dade Behring, Marburg, Deutschland) eingefüllt und 1 Stunde bei +15 bis +25°C inkubiert. Nach weiterem zweimaligen Waschen der Platte wurde in jede Vertiefung 100 µl Chromogen TMB-Lösung (Fa. Dade Behring, Marburg, Deutschland) eingefüllt und weitere 30 Minuten bei +15 bis +25°C inkubiert. Nach der Inkubation wurde in jede Vertiefung 100 µl Stopplösung POD (Fa. Dade Behring, Marburg. Deutschland) eingefüllt und die Mikrotiterplatte am BEP II (Behring-ELISA-Prozessor II, Fa. Dade Behring, Marburg, Deutschland) bei 450 nm ausgewertet.

In einem 2. Testschritt wurden die Hybride nach Vereinzelung wie oben beschrieben noch einmal im gleichen Testformat überprüft.

### d) Klonierung

Einzelne Zellen von Hybriden, die Prothrombin-spezifische Antikörper produzieren, wurden mit einem Mikromanipulator (Fa. Leitz, Wetzlar, Deutschland) kloniert. Kulturüberstände dieser Klone wurden wie unter g) beschrieben gereinigt und wie unter e), h) und i) beschrieben näher charakterisiert. Ein erfindungsgemäßer Antikörper (Bindung an ein Epitop in der N-terminalen Hälfte / Region des humanen Prothrombin-Fragmentes F 2) wird beispielsweise vom Klon 92-195/097 produziert. Diese Hybridomazelllinie wurde bei der DSMZ Deutsche Sammlung von Mikroorganismen und Zellkulturen GmbH, Mascheroder Weg 1b, 38124 Braunschweig, Deutschland, mit der Eingangsnummer DSM ACC2607 hinterlegt.

### e) Bestimmung der Antikörpersubklasse

Die Subklasse des Antikörpers 92-195/097 wurde mittels IsoStrip™-Mouse Monoclonal Antibody Isotyping Kit- der Fa. Boehringer Mannheim, Deutschland als IgG₁ bestimmt.

### f) Produktion der Antikörper

Für die Produktion größerer Mengen Antikörper werden die entsprechenden Zellklone in Rollerflaschen (Fa. Corning Costar Deutschland, Bodenheim) überführt, und bis zum gewünschten Endvolumen bei +37°C expandiert. Danach wird die Rollerkultur-Suspension zur Entfernung der Zellen über 0,22 µm filtriert. Die jetzt zellfreie Antikörperlösung wird über Ultrafilter (Trenngrenze 30 000 Dalton) ankonzentriert und anschließend aufgereinigt.

### g) Reinigung der Antikörper

Die erhaltene Antikörperlösung wird gegen 0,14 M Phosphatpuffer pH 8,6 umgepuffert und auf ein mit rProtein A Sepharose Fast Flow (Fa. Amersham Pharmacia) gefüllte Chromatographiesäule aufgetragen (pro 10 mg zu reinigender Antikörper werden 1 ml rProtein A Sepharose Fast Flow eingesetzt). Alle nicht gebundenen Komponenten werden durch Waschen der Säule mit 0,14 M Phosphatpuffer pH 8,6 entfernt. Der gebundene Antikörper wird mit 0,1 M Zitronensäure pH 3,0 von der Säule eluiert und gegen 0,05 M Natriumacetat + 0,5 M NaCl + 0,05 M Tris + 0,01% Natriumazid pH 7,0 dialysiert.

### h) Selektion geeigneter Antikörper für einen Prothrombin-Fragment F₁₊₂-Sandwich ELISA

Die Reaktion der monoklonalen anti-Prothrombin-Antikörper mit Thrombin und mit F₂ wurde untersucht:

### Reaktion mit Thrombin:

Als Festphase wird eine Mikrotiterplatte verwendet, die mit Kaninchen-anti-Maus-IgG beschichtet ist. Die Anti-Prothrombin-Antikörper aus Kulturüberständen werden daran gekoppelt. Nach einem Waschschritt erfolgt die Inkubation mit gereinigtem Thrombin. Nach einem weiteren Waschschritt wird die Bindung des Antikörpers an Thrombin durch ein Konjugat bestehend aus monoklonalen Anti-Thrombin-Antikörpern von der Maus und dem Enzym Peroxidase mit anschließender Farbreaktion nachgewiesen.

### Reaktion mit F ₂:

Als Festphase wird eine Mikrotiterplatte verwendet, die mit Kaninchen-anti-Maus-IgG beschichtet ist. Die Anti-Prothrombin-Antikörper aus Kulturüberständen werden daran gekoppelt. Nach einem Waschschritt erfolgt die Inkubation mit gereinigtem Prothrombin-Fragment F₂. Nach einem weiteren Waschschritt wird die Bindung des Antikörpers an F₂ durch ein Konjugat bestehend aus polyklonalen Anti-F₂-Antikörpern vom Kaninchen und dem Enzym Peroxidase mit anschließender Farbreaktion nachgewiesen.

Es wurden solche Antikörper selektiert, die eine Reaktion mit F₂ zeigten, gleichzeitig aber nicht mit Thrombin reagierten. Die Eignung dieser Antikörper zur Verwendung als Konjugat in einem Sandwich-ELISA mit F₂- bzw. F₁₊₂-spezifischen Primärantikörpern wurde untersucht. Dazu wurden die gereinigten Antikörper mit einem dem Fachmann bekannten Verfahren an Meerrettich-Peroxidase gekoppelt (Nakane-Kopplung).

Die Überprüfung der Eignung erfolgte im Sandwich ELISA, wie im Beispiel 3a) beschrieben. Die wesentlichen Entscheidungskriterien für die Eignung waren eine optimale Signalstärke, Größe des Messbereiches, untere Nachweisgrenze, und die Linearität der Kalibrationskurve.

Der vom Klon 92-195/097 produzierte Antikörper zeigte hinsichtlich dieser Kriterien die besten Ergebnisse.

### i) Epitopmapping

Das Prothrombinfragment F₁₊₂ wurde in 13-mer-Peptide aufgeteilt, die sich jeweils um 11 Aminosäuren überschneiden und sich dementsprechend in 2-er-Schritten sukzessive vom N-Terminus bis zum C-Terminus fortpflanzen. Diese Peptide wurden synthetisch hergestellt, auf eine Membran gekoppelt und die Bindung des zu analysierenden Antikörpers an jedes dieser Peptide untersucht: Zum Nachweis wurde der zu untersuchende Antikörper zuvor kovalent an Meerrettich-Peroxidase gekoppelt. In einer nachfolgenden Reaktion setzt die Meerrettich-Peroxidase ein Chemilumineszenz-Substrat um, dessen Signal mit Hilfe eines Imaging Systems quantifiziert wird. D.h., je mehr Antikörper an ein bestimmtes Peptid gebunden hat, um so stärker ist das gemessene Signal mit diesem Peptid.

Die Ursache für die geringe Ausbeute bei der Suche nach einem geeigneten Sekundärantikörper ist offenbar, dass zur Kombination mit einem Primärantikörper gegen den freien C-Terminus des Prothrombin F₁₊₂-Fragmentes eine spezifische Bindungsstelle des Sekundärantikörpers bevorzugt in der N-terminalen Hälfte des F₂-Fragmentes erforderlich ist. Insbesondere ist eine spezifische Bindung an die Aminosäuresequenz Ser-Pro-Pro-Leu-Glu-Gln-Cys bevorzugt:

Epitop-Mapping des vom Klon 92-195/097 produzierten Sekundär-Antikörpers, Ergebnis (gezeigt ist ein Ausschnitt aus insgesamt 180 Peptiden, deren Aminosäuresequenz um jeweils 2 Aminosäuren pro Peptid versetzt ist):

| **Peptid** | **Sequenz** | **Signalstärke** |
|---|---|---|
| 100 | Ser-Glu-Gly-Ser-Ser-Val-Asn-Leu-Ser-Pro-Pro-Leu-Glu | 12842 |
| 101 | Gly-Ser-Ser-Val-Asn-Leu-Ser-Pro-Pro-Leu-Glu-Gln-Ser* | **350189** |
| 102 | Ser-Val-Asn-Leu-Ser-Pro-Pro-Leu-Glu-Gln-Ser*-Val-Pro | **406370** |
| 103 | Asn-Leu-Ser-Pro-Pro-Leu-Glu-Gln-Ser*-Val-Pro-Asp-Arg | **434256** |
| 104 | Ser-Pro-Pro-Leu-Glu-Gln-Ser*-Val-Pro-Asp-Arg-Gly-Gln | **538562** |
| 105 | Pro-Leu-Glu-Gln-Ser*-Val-Pro-Asp-Arg-Gly-Gln-Gln-Tyr | **102606** |
| 106 | Glu-Gln-Ser*-Val-Pro-Asp-Arg-Gly-Gln-Gln-Tyr-Gln-Gly | 15952 |
| 107 | Ser*-Val-Pro-Asp-Arg-Gly-Gln-Gln-Tyr-Gln-Gly-Arg-Leu | 14287 |
| 108 | Pro-Asp-Arg-Gly-Gln-Gln-Tyr-Gln-Gly-Arg-Leu-Ala-Val | 13364 |
| 109 | Arg-Gly-Gln-Gln-Tyr-Gln-Gly-Arg-Leu-Ala-Val-Thr-Thr | 13017 |

| | | |
|---|---|---|
| *: Aus technischen Gründen wurde bei der Peptidsynthese Cystein aus der Originalsequenz gegen Serin ausgetauscht. Die Immunreaktvität des Antikörpers auf beide Aminosäuren unterscheidet sich nicht in dieser Methode. Deshalb hat der Austausch keinen Einfluss auf das Endergebnis. | | |

### 3. Beispiel: Nachweis von F ₁₊₂ in einer Probe

### a) Testverfahren

Die erfindungsgemäßen Sekundärantikörper wurden in Kombination mit den Primärantikörpern in einem Enzymimmunoassay nach dem Sandwich-Prinzip eingesetzt:

Während der ersten Inkubation bindet sich das in der Probe vorhandene F₁₊₂-Antigen an die gegen F₁₊₂ gerichteten Primärantikörper, die an der Oberfläche von Vertiefungen einer Mikrotitrationsplatte fixiert sind. Nach Auswaschen der Vertiefungen werden in einer zweiten Reaktion an Peroxidase-konjugierte erfindungsgemäße Sekundärantikörper an die freien F₁₊₂-Determinanten gebunden. Die überschüssigen Enzym-konjugierten Sekundärantikörper werden ausgewaschen. Anschließend wird die gebundene Enzym-Aktivität in den Vertiefungen bestimmt. Die enzymatische Umsetzung von Wasserstoffperoxid und Tetramethylbenzidin wird durch Zusatz von verdünnter Schwefelsäure unterbrochen. Die der Konzentration von F₁₊₂ proportionale Farbintensität wird photometrisch bestimmt und anhand einer Kalibrationskurve aus mitgelieferten Standards quantifiziert.

Ein solcher erfindungsgemäße Sandwich-Immunoassay zeigt hinsichtlich der folgenden Eigenschaften gegenüber bekannten F₁₊₂-Testen verbesserte Resultate:

### b) Testhomogenität:

Die Homogenität einer Probe, gemessen auf 20 Testplatten mit je 96 Bestimmungen, beträgt 5,2 % VK (= Variationskoeffizient).

### c) Linearität des Messbereiches:

Eine lineare Verdünnbarkeit über den gesamten Messbereich ist gegeben:

| **Probe A** | **nmol/L** | **nmol/L x Verdünnung** | **Wiederfindung %** |
|---|---|---|---|
| Unverd. | 3,940 | 3,94 | 100 % |
| 1 : 2 | 2,060 | 4,12 | 105 % |
| 1 : 4 | 0,985 | 3,94 | 100 % |
| 1 : 16 | 0,206 | 3,30 | 84 % |

| **Probe B** | **nmol/L** | **nmol/L x Verdünnung** | **Wiederfindung %** |
|---|---|---|---|
| Unverd. | 0,900 | 0,90 | 100% |
| 1 : 2 | 0,430 | 0.86 | 96 % |
| 1 : 5 | 0,166 | 0,83 | 92 % |
| 1 : 8 | 0.114 | 0,91 | 101 % |
| 1 : 10 | 0,081 | 0,81 | 90 % |
| 1 : 15 | 0,064 | 0,96 | 107 % |

### d) Diagnostische Sensitivität:

Die diagnostische Sensitivität des erfindungsgemäßen Sandwich-Immunoassay ist gegenüber dem Stand der Technik verbessert:

| **Diagnostische Sensitivität*** | **Mit monoklonalen Antikörpern** | **Stand der Technik (kommerziell erhältlicher Test)** |
|---|---|---|
| Bei oral antikoagulierten Patienten (n = 18): | 83 % | 78 % |
| Bei Thrombophilie-Patienten (n =18): | 67 % | 61 % |

| | | |
|---|---|---|
| (*bei 95 % diagnostischer Spezifität; n = 40) | | |

## Patentansprüche

1. Peptid umfassend die Aminosäuresequenz Pro-Leu-Glu-Gln-Cys **dadurch gekennzeichnet, dass** es aus der Aminosäuresequenz Ser-Glu-Gly-Ser-Ser-Val-Asn-Leu-Ser-Pro-Pro-Leu-Glu-Gln-Cys-Val-Pro-Asp-Arg-Gly-Gln-Gln-Tyr-Gln-Gly oder aus der Aminosäuresequenz Ser-Pro-Pro-Leu-Glu-Gln-Cys besteht.

2. Peptid nach Anspruch 1, **dadurch gekennzeichnet, dass** dieses mit einer Festphase und/oder einer Komponente eines signalbildenden Systems assoziiert ist.

3. Verwendung eines Peptides nach Anspruch 1 oder 2 zur Reinigung von Antikörpern in vitro.

4. Verwendung eines Peptids nach einem der Ansprüche 1-3 in einem in vitro-Verfahren zum quantitativen oder qualitativen Nachweis eines Analyten, bevorzugt F₁₊₂.

5. Antikörper, der spezifisch an ein Epitop auf der N-terminalen Hälfte des F₂-Fragmentes von Prothrombin bindet, **dadurch gekennzeichnet**, dieser an ein Peptid gemäß Anspruch 1 spezifisch bindet.

6. Antikörper nach Anspruch 5, **dadurch gekennzeichnet, dass** dieser ein monoklonaler oder ein polyklonaler Antikörper ist.

7. Antikörper nach einem der Ansprüche 5-6, **dadurch gekennzeichnet, dass** dieser von der Hybridomazelllinie DSM ACC2607 produziert wird.

8. Antikörper nach einem der Ansprüche 5-7, **dadurch gekennzeichnet, dass** der Antikörper mit einer Festphase und/oder einer Komponente eines signalbildenden Systems assoziiert ist.

9. Antikörper nach einem der Ansprüche 5-7 in einem pharmazeutisch verträglichen, sterilen Injektionsmedium.

10. Verwendung eines Antikörpers nach einem der Ansprüche 5-8 in einem in vitro-Verfahren zum quantitativen oder qualitativen Nachweis eines Analyten, bevorzugt F₁₊₂.

11. Verwendung eines Antikörpers nach einem der Ansprüche 5-8 in der Affinitätschromatographie.

12. Verwendung eines Antikörpers nach einem der Ansprüche 5-8 als in vitro-Diagnostikum oder als Bestandteil eines in vitro-Diagnostikums.

13. Reagenz enthaltend ein oder mehrere Peptide nach Anspruch 1 und/oder einen oder mehrere Antikörper nach einem der Ansprüche 5-8.

14. Testkit enthaltend ein oder mehrere Peptide nach Anspruch 1 und/oder einen oder mehrere Antikörper nach einem der Ansprüche 5-8 und/oder ein Reagenz gemäß Anspruch 13.

15. Tierische, pflanzliche oder prokaryontische Zelle sowie isolierte menschliche Zelle, **dadurch gekennzeichnet, dass** diese Antikörper nach einem der Ansprüche 5-6 produziert.

16. Hybridomazellinie nach Anspruch 15.

17. Hybridomazelllinie nach Anspruch 16, die bei der DSMZ unter der Eingangsnummer DSM ACC2607 hinterlegt wurde.

18. Verfahren zum quantitativen oder qualitativen Nachweis der ProthrombinFragmente F₁₊₂ und/oder F₂ in einer Probe unter Verwendung eines oder mehrerer Peptide nach Anspruch 1 und/oder eines oder mehrerer Antikörper nach einem der Ansprüche 5-8.

19. Verfahren nach Anspruch 18, **dadurch gekennzeichnet, dass** Antikörper nach einem der Ansprüche 5-8 in Kombination mit Antikörpern, deren Epitope die vier carboxyterminalen Aminosäuren der F₂- und F₁₊₂-Fragmente umfassen, verwendet werden.

## Claims

1. Peptide comprising the amino acid sequence Pro-Leu-Glu-Gln-Cys, **characterized in that** it consists of the amino acid sequence Ser-Glu-Gly-Ser-Ser-Val-Asn-Leu-Ser-Pro-Pro-Leu-Glu-Gln-Cys-Val-Pro-Asp-Arg-Gly-Gln-Gln-Tyr-Gln-Gly or of the amino acid sequence Ser-Pro-Pro-Leu-Glu-Gln-Cys.

2. Peptide according to Claim 1, **characterized in that** it is associated with a solid phase and/or a component of a signal-generating system.

3. Use of a peptide according to Claim 1 or 2 for purifying antibodies in vitro.

4. Use of a peptide according to any of claims 1-3 in an in vitro method for the quantitative or qualitative detection of an analyte, preferably F₁₊₂.

5. Antibody which binds specifically to an epitope on the N-terminal half of the F₂ fragment of prothrombin **characterized in that** it binds specifically to a peptide according to Claim 1.

6. Antibody according to Claim 5, **characterized in that** it is a monoclonal or a polyclonal antibody.

7. Antibody according to any of Claims 5-6, **characterized in that** it is produced by the hybridoma cell line DSM ACC2607.

8. Antibody according to any of Claims 5-7, **characterized in that** it is associated with a solid phase and/or a component of a signal-generating system.

9. Antibody according to any of Claims 5-7 in a pharmaceutically acceptable, sterile injection medium.

10. Use of an antibody according to any of claims 5-8 in an in-vitro method for the quantitative or qualitative detection of an analyte, preferably F₁₊₂.

11. Use of an antibody according to any of Claims 5-8 in affinity chromatography.

12. Use of an antibody according to any of Claims 5-8 as an in-vitro diagnostic aid or as constituent of an in-vitro diagnostic aid.

13. Reagent comprising one or more peptides according to Claim 1 and/or one or more antibodies according to any of Claims 5-8.

14. Test kit comprising one or more peptides according to Claim 1 and/or one or more antibodies according to any of Claims 5-8 and/or a reagent according to Claim 13.

15. Animal, plant or prokaryotic cell, and isolated human cell, **characterized in that** it produces antibodies according to any of Claims 5-6.

16. Hybridoma cell line according to Claim 15.

17. Hybridoma cell line according to Claim 16, which was deposited at the DSMZ under accession number DSM ACC2607.

18. Method for the quantitative or qualitative detection of prothrombin fragments F₁₊₂ and/or F₂ in a sample using one or more peptides according to Claim 1 and/or one or more antibodies according to any of Claims 5-8.

19. Method according to Claim 18, **characterized in that** antibodies according to any of Claims 5-8 are used in combination with antibodies whose epitopes include the four carboxy-terminal amino acids of the F₂ and F₁₊₂ fragments.

## Revendications

1. Peptide comprenant la séquence d'acides aminés Pro-Leu-Glu-Gln-Cys, **caractérisé en ce qu'**il est constitué de la séquence d'acides aminés Ser-Glu-Gly-Ser-Ser-Val-Asn-Leu-Ser-Pro-Pro-Leu-Glu-Gln-Cys-Val-Pro-Asp-Arg-Gly-Gln-Gln-Tyr-Gln-Gly ou de la séquence d'acides aminés Ser-Pro-Pro-Leu-Glu-Gln-Cys.

2. Peptide selon la revendication 1, **caractérisé en ce que** celui-ci est associé à une phase solide et/ou à un composant d'un système générateur d'un signal.

3. Utilisation d'un peptide selon la revendication 1 ou 2, pour la purification d'anticorps in vitro.

4. Utilisation d'un peptide selon l'une quelconque des revendications 1 à 3, dans un procédé in vitro pour la détection quantitative ou qualitative d'un analyte, de préférence, F₁₊₂.

5. Anticorps, qui se lie de manière spécifique à un épitope situé sur la moitié N-terminale du fragment F₂ de la prothrombine, **caractérisé en ce que** celui-ci se lie spécifiquement à un peptide selon la revendication 1.

6. Anticorps selon la revendication 5, **caractérisé en ce que** celui-ci est un anticorps monoclonal ou un anticorps polyclonal.

7. Anticorps selon l'une quelconque des revendications 5 à 6, **caractérisé en ce que** celui-ci est produit à partir de la lignée de cellules d'hybridome DSM ACC2607.

8. Anticorps selon l'une quelconque des revendications 5 à 7, **caractérisé en ce que** l'anticorps est associé à une phase solide et/ou à un composant d'un système générateur d'un signal.

9. Anticorps selon l'une quelconque des revendications 5 à 7, dans un milieu d'injection stérile pharmaceutiquement acceptable.

10. Utilisation d'un anticorps selon l'une quelconque des revendications 5 à 8, dans un procédé in vitro pour la détection quantitative ou qualitative d'un analyte, de préférence, F₁₊₂.

11. Utilisation d'un anticorps selon l'une quelconque des revendications 5 à 8 en chromatographie d'affinité.

12. Utilisation d'un anticorps selon l'une quelconque des revendications 5 à 8, comme diagnostic in vitro ou comme élément d'un diagnostic in vitro.

13. Réactif contenant un ou plusieurs peptides selon la revendication 1 et/ou un ou plusieurs anticorps selon l'une quelconque des revendications 5 à 8.

14. Kit d'essai contenant un ou plusieurs peptides selon la revendication 1 et/ou un ou plusieurs anticorps selon l'une quelconque des revendications 5 à 8 et/ou un réactif selon la revendication 13.

15. Cellule animale, végétale ou procaryote, également, cellule humaine isolée, **caractérisée en ce que** celle-ci produit des anticorps selon l'une quelconque des revendications 5 à 6.

16. Lignée de cellules d'hybridome selon la revendication 15.

17. Lignée de cellules d'hybridome selon la revendication 16, qui a été déposée auprès de la DSMZ sous le numéro d'entrée DSM ACC2607.

18. Procédé de détection quantitative ou qualitative des fragments F₁₊₂ et/ou F₂ de la prothrombine dans un échantillon en utilisant un ou plusieurs peptides selon la revendication 1 et/ou un ou plusieurs anticorps selon l'une quelconque des revendications 5 à 8.

19. Procédé selon la revendication 18, **caractérisé en ce que** l'on utilise des anticorps selon l'une quelconque des revendications 5 à 8 en combinaison avec des anticorps, dont les épitopes comprennent les quatre acides aminés en position carboxy-terminale des fragments F₂ et F₁₊₂.


### SEQUENZPROTOKOLL

<110> Dade Behring Marburg GmbH
<120> Gegen das Prothrombin-Fragment F1+2 gerichtete Antikörper; ihre Herstellung und Verwendung
<130> Mal253
<140>
   <141>
<160> 4
<170> PatentIn Ver. 2.1
<210> 1
   <211> 7
   <212> PRT
   <213> Homo sapiens
<400> 1
   Ser Pro Pro Leu Glu Gln Cys
   1 5
<210> 2
   <211> 5
   <212> PRT
   <213> Homo sapiens
<400> 2
   Pro Leu Glu Gln Cys
   1 5
<210> 3
   <211> 25
   <212> PRT
   <213> Homo sapiens
<400> 3
   Ser Glu Gly Ser Ser Val Asn Leu Ser Pro Pro Leu Glu Gln Cys Val
   1 5 10 15
   Pro Asp Arg Gly Gln Gln Tyr Gln Gly
   20 25
<210> 4
   <211> 62
   <212> PRT
   <213> Homo sapiens
<400> 4
   Ser Glu Gly Ser Ser Val Asn Leu Ser Pro Pro Leu Glu Gln Cys Val
    1               5                   10                  15
   Pro Asp Arg Gly Gln Gln Tyr Gln Gly Arg Leu Ala Val Thr Thr His
               20                  25                  30
   Gly Leu Pro Cys Leu Ala Trp Ala Ser Ala Gln Ala Lys Ala Leu Ser
           35                  40                  45
   Lys His Gln Asp Phe Asn Ser Ala Val Gln Leu Val Glu Asn
       50                  55                  60